# EUROPÄISCHE PATENTSCHRIFT

(11) **EP 0 236 374 B1**
(45) Veröffentlichungstag und Bekanntmachung des Hinweises auf die Patenterteilung: **17.02.1993**
(21) Anmeldenummer: 86905204.3
(22) Anmeldetag: 29.08.1986
(51) Int. Cl.: A61K 7/48

(54) **KOSMETISCHE ZUSAMMENSETZUNG, INSBESONDERE ZU DEKORATIVEN ZWECKEN**
COSMETIC COMPOSITION, IN PARTICULAR FOR ESTHETIC PURPOSES
COMPOSITION COSMETIQUE, EN PARTICULIER A USAGE ESTHETIQUE

(30) Priorität: 29.08.1985 DE 3530902
(43) Veröffentlichungstag der Anmeldung: 16.09.1987
(73) Patentinhaber: Roller, Iris, geb. Zastrau, D-76571 Gaggenau (DE)
(72) Erfinder: Roller, Joachim, W-7560 Gaggenau (DE)
(74) Vertreter: Neidl-Stippler, Cornelia, Dr.
(86) Internationale Anmeldenummer: DE8600347
(87) Internationale Veröffentlichungsnummer: WO8701278

(56) Entgegenhaltungen:
- EP-A- 63 467
- DE-C- 237 624
- FR-A- 1 137 291
- US-A- 4 021 263
- PATENT ABSTRACTS OF JAPAN, Band 10, Nr. 78 (C-335)(2135), 27 März 1986*
- CHEMICAL ABSTRACTS, Band 96, Nr. 2, Januar 1982, Columbus, OH (US); Seite 295, Nr. 24658d*
- CHEMICAL ABSTRACTS, Band 98, 30 Mai 1983, Columbus, OH (US); Seite 359, Nr. 185401w*
- CHEMICAL ABSTRACTS, Band 102, 14 Januar 1985, Columbus, OH (US); Seite 349, Nr. 12218q*
- CHEMICAL ABSTRACTS, Band 48, 10 Januar 1954, Columbus, OH (US); M.TAKAHASHI, Spalte 336-b*
- CHEMICAL ABSTRACTS, Band 86, 13 Juni 1977, Columbus, OH (US); A.N.ROHL et al., Seiten 365-366, Nr. 177164h*

## Beschreibung

Die Erfindung betrifft die Verwendung von Edelsteinpulver für kosmetische Zusammensetzung, insbesondere zu dekorativen Zwecken.

Bei dekorativer Kosmetik handelt es sich meist um mit Farbstoffen oder Farbpigmenten versetzte Trägermaterialien, welche bei Trägerinnen Farbakzente im Gesicht, auf der Frisur u.s.w. setzen sollen. Derartige Kosmetika mit Edelsteinen und edelsteinartigen Materialien sind vewreinzelt aus EP-A-0063467, DE-C-237624, US-A-4,021,263 sowie aus den Chemical Abstracts, Band 48, Spalte 336-b (1954); Band 98, Nr. 185401 w (1983) und Band 102, Nr. 12218 q (1985) bekannt. Typische dekorative Kosmetika sind Nagellacke, Lidschatten, Lippenstifte, Puder - sowohl Gesichts- als auch Körperpuder, Haarspray, und so fort.

Bei den Zusammensetzungen treten bei den Trägerinnen in neuerer Zeit in hohem Maße allergische Reaktionen auf, insbesondere wenn Farbstoffe oder Trägermaterialien eingesetzt werden, die sich bei längerem Stehenlassen als Bakteriennährböden, zur Ansiedelung von Mikroorganismen eignen oder durch einfachen chemischen Zerfall - beispielsweise durch Lichteinwirkung - altern - und die Zerfallsprodukte Allergien hervorrufen.

Ein weiterer Nachteil besteht bei bekannten dekorativen Kosmetika darin, daß sie nur eine sehr begrenzte Verbrauchsdauer haben und häufig temperaturempfindlich sind; demzufolge ist die Lagerhaltung in Kosmetikfachgeschäften nicht problemlos.

Auch umfangreiche Tierversuche vermögen derartige allergische Reaktionen, wie sie insbesondere bei einem durch längeren bzw. unsachgemäße Lagerung entstehenden Zerfall hervorgerufen werden, nicht zweifelsfrei vorauszusagen.

Ferner besteht bei Abendkosmetik oder auch Schauspieler-Schminken ein steigendes Bedürfnis an besonders brillianten, reflektierenden Farben, wobei die Anforderungen an Brillianz und hohes Reflektionsvermögen in diesem Bereich von üblichen organischen Farbstoffen oder auch normalen anorganischen Mineralien nicht erfüllt werden.

Es ist daher Aufgabe der Erfindung, eine verbesserte kosmetische Zusammensetzung zu schaffen, welche bei besseren optischen Wirkungen verträglichere Farbstoffe einsetzt.

Diese Aufgabe wird erfindungsgemäß gelöst durch die Verwendung von reinem Edelsteinpulver einer Korngrößenverteilung zwischen 0,0005 und 0,003 mm als allergenschwacher Farbstoff in dekorativen hautkosmetischen Zusammensetzungen, ausgenommen Nagellacken, Zahnpasten, wobei das Pulver von Edelsteinen oder Edelsteinmischungen stammt, die ausgewählt sind aus der Gruppe bestehend aus Diamant, Saphir, Rubin, Smaragd, Edeltopas, Mondstein, Katzenauge, Zirkon, Bergkristall, Achat, Amethyst, Aquamarin, Aventurin, Azurit, Bernstein, Beryll, Chrysoberyll, Chrysophras, Chrysolit, Chrysokoll, Chalzedon, Dioptas, Feueropal, Fluorit, Granat, Goldtopas, Goldberyll, Hyazinth, Hämatit, Jade, Karneol, Lapislazuli, Labradorit, Malachit, Nephrit, Onyx, Opal, Obsidian, Padpardsha, Peridot, Rhodolit, Rhodochrosit, Rubelit, Saphirquartz, Sodalit, Spinell, Sard-Onyx, Tansanit, Topas, Turmalin, Tigerauge, Zoisit, Türkis, Chile-Lapis, Quartz, Rauchquartz, Jaspis und Korallen. Bevorzugte Ausführungsformen der Erfindung sind in den Unteransprüchen beschrieben.

Der Edelsteinfarbstoff wird bevorzugt zusammen mit einem flüssigen, hochviskosen oder festen, hautverträglichen Trägermaterial eingesetzt. Der Farbstoff ist ein reines Pulver von Edelsteinen oder Edelsteinmischungen mit der erfindungsgemäßen Korngrößenverteilung.

Die Zusammensetzung kann selbstverständlich, wie bei Kosmetika üblich, Duftstoffe enthalten.

Bei einer bevorzugten Ausführungsform wird Edelsteinpulver in eine Trägerflüssigkeit eingebracht, in welcher es - gegebenenfalls unter Zuhilfenahme von Emulsionsstabilisatoren - emulgiert oder emulgierbar ist. Derartige Flüssigkeiten eignen sich beispielsweise als flüssige Lidschatten, flüssige Lip-Gloss, Nagellacke und ähnliches.

Für andere bevorzugte Anwendungszwecke hat es sich als günstig erwiesen, die erfindungsgemäße Zusammensetzung in einem hochviskosen, salbenartigen Trägermaterial einzusetzen, wie es beispielsweise für Creme-Lidschatten, weiche Lippenstifte, Lip-Gloss-Creme und ähnliches erwünscht ist. Dabei hat sich herausgestellt, daß sich Eucerin oder Vaseline besondersgutals stabiles Trägermaterial eignet.

Als zusätzliches Hilfsmittel kann auch noch Kieselsäure zugesetzt werden.

Eine weitere bevorzugte Ausführungsform der erfindungsge mäßen Zusammensetzung ist eine Mischung von Edelsteinpulver mit an sich bekannten Pudergrundlagen, wie es beispielsweise Talkum ist. Diese Puder können als Glanz- Make-up bzw. Glanz-Körperpuder eingesetzt werden - entweder in loser oder in verpreßter Form.

Die erfindungsgemäßen dekorativen Zusammensetzungen enthalten das Edelsteinpulver in Mengen um bis zu 10 Gew%, bevorzugt um etwa 5 Gew%. Abhängig vom Einsatzzweck kann die Menge jedoch auch erhöht oder erniedrigt werden.

Dadurch, daß als Farbstoffe reine mineralische kristallisierte Verbindungen eingesetzt werden, welche zudem sehr hart sind und keine Bakteriennährböden zu bilden vermögen aufgrund der fehlenden Poren der Oberfläche - können allergische Reaktionen auf diesen farbgebenden Materialien praktisch ausgeschlossen werden.

Bisher verwendete Erdfarbstoffe wie Tone, sind aufgrund ihrer porösen Oberfläche ein geradezu idealer Nährboden für Bakterien.

Aber auch organische Farbstoffe, die z.B. gemeinsam mit Metallpulvern für Lidschatten verwendet werden, führten häufig zu allergischen Reaktionen gegen die Farbstoffe selbst oder Reaktionsnebenprodukte, die ihnen anhafteten. Als "Edelsteine" haben sich bisher folgende Materialien bewährt: Diamant, Saphir, Rubin, Smaragd, Edeltopas, Mondstein, Katzenauge, Zirkon, Bergkristall, Achat, Amethyst, Aquamarin, Aventurin, Bernstein, Beryll, Chrysoberyll, Chrysophras, Chrysolit, Chrysokoll, Chalzedon, Dioptas, Feueropal, Fluorit, Granat, Goldtopas, Goldberyll, Hyazinth, Hämatit, Jade, Karneol, Lapislazuli, Labradorit, Malachit, Nephrit, Onyx, Opal, Obsidian, Padpardsha, Peridot, Rhodolit, Rhodochrosit, Rubelit, Saphirquarz, Sodalit, Spinell, Sard-Onyx, Tansanit, Topas, Turmalin, Tigerauge, Zoisit, Türkis, Chile-Lapis, Quarz, Rauchquarz und Jaspis, sowie die in den nachstehenden Beispielen genannten Edelsteine und Edelsteinmischungen.

Zusätzlich zur guten Verträglichkeit zeichnet sich die erfindungsgemäße dekorative Kosmetik durch eine extreme Farbreinheit, Glanzfähigkeit und Brillanz aus, wie sie insbesondere bei der heutigen häufigen Verwendung von Bühnenlasern für Schauspieler-Schminke gefragt ist.

Im folgenden wird die Erfindung anhand von Beispielen näher erläutert:

### Beispiel 1:

### Pulver-Lidschatten

100g Malachit wurden in einer Edelsteinmühle, einer im Handel erhältlichen Achatmühle, 4 Stunden lang zerkleinert. Das derart hergestellte Edelsteinpulver wies eine Korngrößenverteilung zwischen 0,0005 bis 0,003 Millimeter = 0,5 bis 3 µm auf. 5 g des Malachitpulvers wurden gemeinsam mit 95 g Eucerin cum aqua innig in einer Knetmaschine verknetet und die resultierende Mischung in Tiegel abgefüllt. Es ergab sich ein sehr gut haftender, stark leuchtender und reflektierender grüner Lidschatten.

### Beispiel 2

10g Lapis wurden, wie in Beispiel 1 beschrieben, in Pulverform zerkleinert und mit 90 g Vaseline in einer Knetmaschine verknetet. Die resultierende Mischung wurde in Tiegel abgefüllt und lieferte einen leuchtenden Lidschatten von blauer Farbe.

### Beispiel 3

### Puderlidschatten

80g feinpulverisiertes Talkum, 10g Siliziumdioxid, hochrein, sowie 10g gemahlener Türkis wurden in einem Mischer innig vermischt. Anschließend wurde das Pulvergemisch zu einem "Puderstein" verpreßt. Es ergab sich ein intensivfarbiger, türkisfarbener Lidschatten.

### Beispiel 4

### Körperpuder

90g Talkum sowie 5 g zerkleinerte Korallen und 5 g Siliciumdioxid wurden innig vermischt und lose als engelshautfarbenes Körperpuder abgefüllt.

### Beispiel 5

10g Lapis, 10g Saphir, 10g Türkis- und 70g Azurit-Pulver wurden innig mit 100g Vaselin in einer Knetmaschine vermischt. Es ergab sich eine intensive hellblaue Lidschattencreme, die in Cremetöpfchen abgefüllt fast unbegrenzt haltbar war.

## Patentansprüche

1. Verwendung von reinem Edelsteinpulver einer Korngrößenverteilung zwischen 0,0005 bis 0,003 mm als allergenschwacher Farbstoff in dekorativen hautkosmetischen Zusammensetzungen, ausgenommen Nagellacken, Zahnpasten, wobei das Pulver von Edelsteinen oder Edelsteinmischungen stammt, die ausgewählt sind aus der Gruppe bestehend aus Diamant, Saphir, Rubin, Smaragd, Edeltopas, Mondstein, Katzenauge, Zirkon, Bergkristall, Achat, Amethyst, Aquamarin, Aventurin, Azurit, Bernstein, Beryll, Chrysoberyll, Chrysophras, Chrysolit, Chrysokoll, Chalzedon, Dioptas, Feueropal, Fluorit, Granat, Goldtopas, Goldberyll, Hyazinth, Hämatit, Jade, Karneol, Lapislazuli, Labradorit, Malachit, Nephrit, Onyx, Opal, Obsidian, Padpardsha, Peridot, Rhodolit, Rhodochrosit, Rubelit, Saphirquartz, Sodalit, Spinell, Sard-Onyx, Tansanit, Topas, Turmalin, Tigerauge, Zoisit, Türkis, Chile-Lapis, Quartz, Rauchquartz, Jaspis und Korallen.

2. Verwendung gemäß Anspruch 1, dadurch gekennzeichnet, daß bis 10 Gew.% Edelsteinpulver zugegeben wird.

3. Verwendung gemäß Anspruch 1 oder 2, dadurch gekennzeichnet, daß zwischen 2 und 8 Gew.% Edelsteinpulver zugegeben wird.

4. Verwendung gemäß einem der Ansprüche 1 bis 3, dadurch gekennzeichnet, daß 5 Gew%. Edelsteinpulver zugegeben wird.

5. Verwendung gemäß einem der Ansprüche 1 bis 4, dadurch gekennzeichnet, daß das Edelsteinpulver einem hautverträglichen, flüssigen, hochviskosen oder festen Trägermaterial zugesetzt wird.

6. Verwendung gemäß Anspruch 5, dadurch gekennzeichnet, daß das Trägermaterial flüssig, leicht trocknend ist und das Edelsteinpulver in der Flüssigkeit emulgiert, gegebenenfalls unter Zusatz von Emulsionsstabilisatoren, wird.

7. Verwendung gemäß Anspruch 5, dadurch gekennzeichnet, daß das Trägermaterial Eucerin, Vaseline ist.

8. Verwendung gemäß Anspruch 5, dadurch gekennzeichnet, daß das Trägermaterial Talkum, Gips in Pulverform ist.

9. Verwendung gemäß Anspruch 8, dadurch gekennzeichnet, daß das Trägermaterial mit dem Edelsteinpulver nach Vermischung gepreßt wird.

10. Verwendung gemäß einem der Ansprüche 1 bis 9 bei der Herstellung von Make-up, Lidschatten, Lippencremes, Schminke, Gloss, Körperpuder.

## Claims

1. Use of pure precious-stone powder having a grain-size distribution between 0̸.0̸0̸0̸5 and 0̸.0̸0̸3 mm as low-allergy dye in decorative skin-cosmetic compositions, except nail varnishes, toothpastes, wherein the powder originates from precious stones or a mixture of precious stones, which are selected from a group comprising the following: diamond, sapphire, ruby, emerald, precious topas, moonstone, cat's eye, zircon, rock crystal, agate, amethyst, aquamarine, aventurine, azurite, amber, beryl, chrysoberyl, chrysoprase, chrysolite, chrysocolla, chalcedony, dioptase, fire opal, fluorite, garnet, oriental topaz, gold beryl, hyazinth, hematite, jade, cornelian, lapis lazuli, labradorite, malachite, nephrite, onyx, opal, obsidian, padpardsha, peridot, rhodolite, rhodochrosite, rubellite, sapphire quartz, sodalite, spinel, sard onyx, tansanite, topas, turmalin, tigers eye, zoisite, turquoise, chile lapis, quartz, smoky quartz, jaspis and corals.

2. Use according to claim 1, **characterized in that** up to 10̸ weight-% of precious-stone powder is added.

3. Use according to claim 1 or 2, **characterized in that** between 2 and 8 weight-% precious-stone powder are added.

4. Use according to one of claims 1 to 3, **characterized in that** 5 weight-% precious-stone powder are added.

5. Use according to one of claims 1 to 4, **characterized in that** the precious-stone powder is added to a skin-friendly, liquid, highly viscous or solid carrier material.

6. Use according to claim 5, **characterized in that** the carrier material is liquid, easy-drying, and that the precious-stone powder is emulsified in the liquid, if appropriate with the addition of emulsification stabilizers.

7. Use according to claim 5, **characterized in that** the carrier material is Eucerin, Vaseline.

8. Use according to claim 5, **characterized in that** the carrier material is pulverized talcum, gypsum.

9. Use according to claim 8, **characterized in that** after having been mixed, the carrier material is compressed with the precious-stone powder.

10. Use according to one of claims 1 to 9 in the manufacture of makeup, lid shadows, lip cremes, makeup paint, gloss, body powder.

## Revendications

1. Emploi de poudre pure de gemme(s) d'une granulométrie comprise entre 0,0005 et 0,003 comme colorant pauvre en allergène dans des compositions cosmétiques décoratives pour la peau, à l'exception des vernis à ongles et des pâtes dentifrices, emploi dans lequel la poudre provient de gemmes ou de mélanges de gemmes qui sont choisies dans le groupe suivant : diamant, saphir, rubis, émeraude, topaze fine, pierre de lune, oeil de chat, zircon, cristal de roche, agate, améthyste, aigue-marine, aventurine, ambre jaune, béryl, chrysobéryl, chrysoprase, chrysolite, chrysocolle, calcédoine, dioptase, opale de feu, fluorite, grenat, topaze brûlée, béryl doré, hyacinthe, hématite, jade, carnéol, lapis-lazuli, labradorite, malachite, néphrite, onyx, opale, obsidienne, padpardsha, olivine, rhodolite, rhodochrosite, rubélite, quart saphyr, sodalite, spinelle, sardoine, tansanite, topaze, tourmaline, oeil de tigre, zoïsite, turquoise, lapis du Chili, quarts, quarts fumé, jaspe et corail.

2. Emploi selon la revendication 1, caractérisé en ce que l'on ajoute jusqu'à 10 % en poids de poudre de gemme(s).

3. Emploi selon la revendication 1 ou 2, caractérisé en ce que l'on ajoute entre 2 et 8 % en poids de poudre de gemme(s).

4. Emploi selon l'une quelconque des revendications 1 à 3, caractérisé en ce que l'on ajoute 5 % en poids de poudre de gemme(s).

5. Emploi selon l'une quelconque des revendications 1 à 4, caractérisé en ce que la poudre de gemme(s) est ajouté à une matière véhiculaire liquide, très visqueuse ou solide compatible avec la peau.

6. Emploi selon la revendication 5, caractérisé en ce que la matière véhiculaire est liquide et aisément siccative et la poudre de gemme(s) est émulsionnée dans le liquide, éventuellement avec addition de stabilisateurs d'émulsion.

7. Emploi selon la revendication 5, caractérisé en ce que la matière véhiculaire est de l'eucérine ou de la vaseline.

8. Emploi selon la revendication 5, caractérisé en ce que la matière véhiculaire est du talc ou du gypse sous forme pulvérulente.

9. Emploi selon la revendication 8, caractérisé en ce que la matière véhiculaire est comprimée avec la poudre de gemme(s) après mélange.

10. Emploi selon l'une quelconque des revendications 1 à 9 pour la préparation de make-up, d'ombres à paupières, de crèmes à lèvres, de fards, de lustres et de talc.
